# EUROPEAN PATENT APPLICATION

(11) **EP 4 768 917 A1**
(43) Date of publication of application: **01.07.2026**
(21) Application number: 24856521.0
(22) Date of filing: 22.08.2024
(51) Int. Cl.: G01N 33/68, G01N 33/543, G01N 33/574

(54) **CANCER EXAMINATION SUPPORT METHOD AND EXAMINATION KIT**

(30) Priority: 23.08.2023 JP 2023135319
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: KANDA Mitsuro, Nagoya-shi, Aichi 464-8601 (JP)
(74) Representative: Heyerhoff Geiger GmbH & Co. KG
(86) International application number: PCT/JP2024/029843
(87) International publication number: WO 2025/041833

(57) **Abstract**

It was found that SDF4 in a body fluid sample can be a marker for detecting a cancer patient with good sensitivity and specificity. By using a reagent for specifically detecting the SDF4, it is possible to detect gastric cancer, breast cancer, colorectal cancer, pancreatic cancer, esophageal cancer, hepatic cancer, liposarcoma, bladder cancer, brain tumor, head and neck cancer, gallbladder cancer, ovarian cancer, tongue cancer, or uterine cancer. The measurement of the SDF4 in the body fluid sample is a test method capable of detecting various types of cancer with a single test, and can be used for mass screening for cancer. Further, it is capable of detecting even cancer of an early stage with good sensitivity and specificity, and thus is capable of detecting cancer at an early stage to start a treatment.

## Description

### Technical Field

The present invention relates to a biomarker capable of detecting cancer from a body fluid sample. The present invention relates to a biomarker capable of detecting multiple types of cancer at an early stage rather than cancer specific for an organ.

### Background Art

The number of patients of cancer and the number of deaths related to cancer are increasing every year. According to a statistical research conducted across 185 countries in the world by the International Agency for Research on Cancer (IARC) of the World Health Organization (WHO), it is estimated that approximately 18.1 million people newly developed cancer and approximately 9.6 million people died from cancer worldwide in 2018. Considering that the number of patients of cancer was approximately 10.1 million and the number of deaths related to cancer was 6.7 million in 2002, it is evident that both the number of patients of cancer and the number of deaths related to cancer are increasing.

In order to reduce the number of deaths related to cancer, it is very important to detect cancer at an early stage, because the likelihood of achieving remission is increased by detecting cancer at an early stage and starting a treatment. For example, gastrointestinal cancers such as gastric cancer and colorectal cancer have high mortality rates in the world. Examples of a standard method for screening and diagnosing gastrointestinal cancers include endoscopic test, but it has disadvantages such as being invasive, carrying a high risk of complications, requiring time for tissue evaluation, and being costly.

In recent years, it has been attempted to analyze a body fluid, such as blood, for nucleic acids and proteins contained in exosomes, a circulating tumor DNA in the blood, a serum non-coding RNA, and the like by liquid biopsy to detect cancer. Some of these methods can detect cancer with high sensitivity, but they are generally complex in terms of the measurement method and expensive, and thus their clinical application remains very limited. In addition, in cancer screening, it is desirable that the test be able to detect a wide variety of cancer types, rather than detecting only cancer of a specific organ, such as gastric cancer. In particular, it is desirable that the test be capable of detecting cancer for which the number of patients is large, such as colorectal cancer, gastric cancer, pancreatic cancer, and liver cancer.

A biomarker test using an immunological technique, such as ELISA using a blood sample, which has been performed in the art, is suitable for cancer screening in a medical checkup or the like, in that the sample can be collected easily, the test is comparatively less invasive, and requires low costs. However, conventional serum tumor markers such as carcinoembryonic antigen (CEA) and CA19-9 have low sensitivity and specificity and therefore do not provide satisfactory early detection. Accordingly, there is a need for biomarkers that can be used for large-scale screening of early-stage cancer, particularly solid cancer. Patent Literature 1 discloses olfactomedin 4 and Reg IV proteins as serum tumor markers for gastrointestinal cancer, particularly gastric cancer. Although the markers described in Patent Literature 1 exhibit different expression in a patient with gastric cancer in an RT-PCR analysis, they do not show a significant difference in ELISA and have not been put into practical use.

### Citation List

### Patent Literature

Patent Literature 1: Japanese Patent Laid-Open No. 2009-168526

### Non Patent Literature

Non Patent Literature 1: Loei H., et al., J. Proteome Res. 2012, Vol. 11, pp. 1759-1772.
Non Patent Literature 2: Feizi A., et al., Database (Oxford), 2015, 2015: bav051
Non Patent Literature 3: Tomczak K., et al., Contemp. Oncol., 2015, 19: A68-77.
Non Patent Literature 4: Gronborg M., et al., Mol Cell Proteomics, 2006, 5: 157-171.
Non Patent Literature 5: Luo J., et al., Int J Biol Sci. 2016; 12(6): 677-687.

### Summary of Invention

### Technical Problem

The present invention aims to provide a test method and a test kit that can be used in mass screening such as a medical checkup or a comprehensive physical examination. When conducting a cancer test in a medical checkup or a comprehensive physical health examination, it is desirable that a sample can be collected with a low burden on the body and that the test is simple without requiring complicated operations. Collection of a body fluid such as blood or saliva is less invasive, and blood samples are also used for other tests in a medical checkup and the like, so they are suitable for use in mass screening for cancer. As described above, conventional markers such as CEA and CA19-9 have been used, but if there is a biomarker with higher sensitivity and specificity that can detect cancer at an early stage, a more effective test method could be developed. Furthermore, it is more desirable that a test uses a biomarker that is not for an organ specific cancer but can detect multiple types of cancer, because multiple types of cancer can be detected with a single marker. The present invention aims to identify a protein marker in the blood that is capable of detecting cancer at an early stage and to provide a test method and a test kit. Particularly, the present invention aims to identify a marker capable of detecting multiple types of cancer at an early stage, rather than detecting only an organ-specific cancer.

### Solution to Problem

The present invention relates to a test method and a test kit as identified below.
(1) A test support method or a test method including measuring a concentration of SDF4 in a body fluid of a subject, and determining the subject as having cancer when the concentration is higher than a predetermined value.
   The present inventor has found that the concentration of the SDF4 in a body fluid is increased in a patient with cancer. Specifically, it has been found that the concentration of the SDF4 in the body fluid is higher in a patient with cancer than in a healthy individual. Since the test can be performed using a body fluid sample, it can be utilized for identifying cancer through mass screening, such as a medical checkup.
(2) The method according to (1), wherein the subject is identified as having cancer using a criterion that the subject is highly likely to have cancer when a level of the SDF4 in the body fluid is higher than the level in a healthy individual.
   The concentration of the SDF4 in a patient with cancer was higher as compared to that in a healthy individual. Therefore, screening for a patient with cancer can be performed by setting a predetermined value as a reference value.
(3) The method according to (1) or (2), wherein the body fluid is blood.
(4) The method according to (3), wherein the blood is selected from whole blood, serum, and plasma.
   The present method is a less invasive method that enables cancer determination by using a sample derived from a body fluid, specifically blood, as shown in the Examples. The SDF4 is a very excellent marker that can be detected in the blood.
(5) The method according to any one of (1) to (4), wherein the concentration of the SDF4 in the body fluid is measured by an immunoassay.
   A test using an antibody, such as an immunoassay including ELISA, is routinely used in clinical settings. Therefore, the present method can be performed immediately in clinical settings without requiring special equipment or new techniques.
(6) The method according to any one of (1) to (5), wherein the cancer is gastric cancer, breast cancer, colorectal cancer, pancreatic cancer, esophageal cancer, liver cancer, liposarcoma, bladder cancer, brain tumor, head and neck cancer, gallbladder cancer, ovarian cancer, tongue cancer, or uterine cancer.
   The present inventor has revealed that an increased level of the SDF4 in the blood correlates with the onset of multiple types of cancer. In particular, the test for the SDF4 enables detection of multiple types of cancer for which the number of patients is large, such as gastric cancer, breast cancer, and colorectal cancer, as well as pancreatic cancer and the like, which is difficult to detect. Therefore, the present method is very useful, in particular for gastric cancer.
(7) The method according to any one of (1) to (6), wherein the cancer is early-stage cancer.
   The method has been demonstrated to detect even early-stage cancer with good sensitivity and specificity. Cancer is highly likely to be cured when detected and treated at an early stage. Therefore, it is believed that the present method contributes to reduction of the number of deaths related to cancer.
(8) The method according to (7), wherein the early-stage cancer is early-stage gastric cancer.
   As shown in the Examples described below, the present method has been shown to detect early-stage gastric cancer at Stage I with very high sensitivity and specificity. When gastric cancer is detected at an early stage, it results in a very good treatment outcome. Therefore, the present method is an effective method of screening for gastric cancer.
(9) A method of acquiring data for diagnosing cancer, the method including a step of measuring a concentration of SDF4 in a body fluid of a subject.
(10) The method according to (9), wherein the body fluid is blood and is specifically selected from whole blood, serum, and plasma.

The data acquired by measuring the concentration of the SDF4 in the body fluid can be used for diagnosing cancer. Since the data can be acquired using the body fluid sample, it can be utilized for diagnosis of cancer through mass screening, such as a medical checkup.

The data acquisition described above can be performed by an immunoassay. Further, the types of cancer that can be detected thereby includes gastric cancer, breast cancer, colorectal cancer, pancreatic cancer, esophageal cancer, liver cancer, liposarcoma, bladder cancer, brain tumor, head and neck cancer, gallbladder cancer, ovarian cancer, tongue cancer, and uterine cancer. The present method is useful for early-stage cancer, in particular for early-stage gastric cancer.

(11) A cancer marker including SDF4 present in a body fluid, the marker exhibiting upregulated expression in a body fluid of a patient with cancer and serving as a cancer diagnostic marker.

The SDF4 can be detected in the body fluid, and is capable of detecting various types of cancer, including gastric cancer, breast cancer, colorectal cancer, pancreatic cancer, esophageal cancer, liver cancer, liposarcoma, bladder cancer, brain tumor, head and neck cancer, gallbladder cancer, ovarian cancer, tongue cancer, and uterine cancer. Therefore, the SDF4 is a very useful cancer marker.

(12) A test kit for detecting cancer using a body fluid of a subject as a sample, the kit including a reagent that specifically binds to SDF4 and a detection reagent.

The present kit can detect cancer by using the body fluid obtained by a less invasive method of collecting a sample, and the reagent that specifically detects the SDF4. The detection method using the specifically binding reagent is a simple method commonly used in clinical settings, such as an immunoassay, and does not newly require expensive equipment and the like. Therefore, the present kit can be utilized immediately in clinical settings.

(13) The test kit according to (12), wherein the cancer is gastric cancer, breast cancer, colorectal cancer, pancreatic cancer, esophageal cancer, liver cancer, liposarcoma, bladder cancer, brain tumor, head and neck cancer, gallbladder cancer, ovarian cancer, tongue cancer, or uterine cancer.

Since multiple types of cancer can be detected with a single test kit, the present kit is very useful in mass screening for cancer. By using the present kit, early-stage cancer (in particular early-stage gastric cancer) can be detected.

(14) The test kit according to (12) or (13), wherein the reagent that specifically binds to the SDF4 is an antibody.

The antibody may be either a monoclonal antibody or a polyclonal antibody, as long as it can specifically detect the SDF4. A monoclonal antibody is more preferred, because it makes it easier to perform a test while setting a cutoff value.

(15) The test kit according to (14), wherein the detection is performed by ELISA.

ELISA has been conventionally used in clinical settings, and is simple and capable of realizing a test with high sensitivity. Further, it can rapidly process a large number of samples and give results.

(16) A method of diagnosing cancer, the method including the steps of: collecting a body fluid from a subject; measuring a concentration of SDF4 in the body fluid; and comparing the concentration of the SDF4 in the subject with a reference value obtained based on a concentration of the SDF4 in a body fluid of healthy individuals, and determining the subject as being highly likely to have cancer when the concentration in the subject is higher than the reference value.

### Brief Description of Drawings

[Figure 1A] Figure 1A is a graph showing serum SDF4 levels in patients with various types of cancer.
[Figure 1B] Figure 1B is a graph showing serum SDF4 levels in patients with gastric cancer at each disease stage.
[Figure 1C] Figure 1C is a graph showing ROC analysis results of the SDF levels in patients with gastric cancer and healthy individuals.
[Figure 1D] Figure 1D is a graph showing a comparison of performance as a diagnostic marker between the SDF4 and conventionally used markers CEA and CA19-9.
[Figure 1E] Figure 1E is a graph showing the serum SDF4 concentrations in patients with gastric cancer at each disease stage, stratified by sex.
[Figure 1F] Figure 1F is a graph showing ROC analysis results of SDF levels in patients with Stage I gastric cancer and healthy individuals.
[Figure 1G] Figure 1G is a graph showing a comparison of performance as a diagnostic marker between the SDF4 and conventionally used markers CEA and CA19-9 in patients with Stage I gastric cancer.
[Figure 2] Figure 2 is a graph showing overall survival (OS) according to SDF4 levels in patients with advanced gastric cancer.
[Figure 3A] Figure 3A is a graph showing analysis results of SDF4 concentrations in cell lysates and conditioned media of gastric cancer cell lines and normal intestinal epithelial cell lines.
[Figure 3B] Figure 3B is a graph showing the correlation between the SDF4 levels in the conditioned media and the cell lysates.
[Figure 4A] Figure 4A illustrates immunohistochemical images showing SDF4 in a cancer tissue and adjacent normal tissues. Representative images for each disease stage are shown.
[Figure 4B] Figure 4B is a graph showing the SDF4 positive rate in cancer tissue at each stage.
[Figure 5] Figure 5 illustrates a graph showing the serum SDF4 concentrations before and after surgery in patients without gastric cancer recurrence (left) and the serum SDF4 concentrations before and after total gastrectomy (right).

### Description of Embodiment

The present inventor considered that a test performed for the purpose of mass screening for cancer required that collection of a sample be less invasive, and that the test be simple and require low cost. Therefore, the present inventor decided to search for a biomarker present in blood, among body fluids, because it has been considered an immunological test, such as ELISA conventionally used for diagnosis in clinical settings, would satisfy the above requirements.

In this context, the term "body fluid" refers to blood as illustrated in the Examples described herein, as well as lymph fluid, saliva, sweat, urine, and the like. Any body fluid may be used, but a blood sample, which is commonly used for mass screening, is preferably used. The term "blood sample" refers to whole blood, serum, or plasma. The test method refers to a test commonly for detecting a protein in a body fluid, specifically immunoassays (immunological measurement method) such as ELISA, Western blotting, immunofluorescence, immunochemiluminescence, or radioimmunoassay. Among them, ELISA is preferred due to its simplicity and frequent use in clinical settings. Alternatively, a specific binding reagent other than an antibody, such as an antibody mimetic, may be used to construct a similar detection assay.

The test kit may include, in addition to a reagent that specifically binds to the SDF4 and a reagent for detection, an instrument for collecting a body fluid, an instruction manual, and the like. As the detection reagents, for example in the case of ELISA, reagents commonly used in the field, such as colorimetric, fluorescent, or chemiluminescent reagents, may be included.

Hereinafter, the present invention will be described with reference to the data.

To search for a cancer marker present in the body fluid, candidate proteins were selected from a secretome analysis dataset using a gastric cancer cell line (Non Patent Literature 1). From this dataset, 188 secreted protein candidates were obtained. Next, using the Human Cancer Secretome Database (HCSD) (Non Patent Literature 2), it was confirmed that 152 of these proteins were present in the body fluid. Furthermore, it was analyzed whether the expression levels of these proteins were increased in a gastric cancer tissue as compared to normal gastric mucosa using the Cancer Genome Atlas (TCGA) database (Non Patent Literature 3), and 79 candidate proteins were selected. Among these proteins, ten proteins were selected, and concentrations of the ten candidate proteins in the blood were measured in patients with gastric cancer and healthy individuals. As a result, it was found that the SDF4 (stromal cell-derived factor 4) was most suitable for diagnosis of gastric cancer.

The SDF4 is a protein also known as Cab45, and has six EF-hand domains that serve as calcium binding motifs and are said to be involved in Ca²⁺-dependent secretion. It is known that three isoforms exist due to alternative splicing: Cab45-G, which mainly localizes to the Golgi apparatus; Cab45-C, which localizes to the cytoplasm; and Cab45-S, which is secreted. There have been reports suggesting association with cancer, but its function has not been fully elucidated. Non Patent Literature 4 discloses that the SDF4 was included among markers identified using the SILAC method in a pancreatic cancer cell line, and that immunohistochemical staining showed expression of the SDF4 in the cytoplasm in nine of ten cases. Non Patent Literature 5 describes that Cab45-G, an isoform localized to the Golgi apparatus, enhances cell migration, and that a correlation between Cab45-G expression and MMP-2 expression was observed by immunohistochemical staining in uterine cervical cancer and esophageal cancer.

However, in each of the above reports, expression was confirmed only within a tissue by immunostaining, and there is no disclosure that the SDF4 can be detected in a body fluid sample such as blood. None of the literatures describe a relationship between the secreted isoform Cab45-S and cancer, and it was unknown whether the SDF4 can be used in a test using a body fluid sample. In addition, the immunohistochemical tests described in Non Patent Literatures 4 and 5 are performed after collecting a tissue sample suspected of cancer, and therefore are not suitable for a test intended for mass screening. As described above, since the purpose of the present invention is to identify a cancer marker to be used in mass screening, a test using a body fluid such as blood, rather than an immunohistochemical test, is required.

Accordingly, the present inventor analyzed the correlation between the SDF4 in a blood sample and cancer. The analysis was performed using the following patient samples. Serum samples obtained from a total of 582 individuals, including patients with gastric cancer (N = 396), breast cancer (N = 30), colorectal cancer (N = 30), pancreatic cancer (N = 27), esophageal cancer (N = 75), and liver cancer (N = 24), diagnosed between 2014 and 2020 at Nagoya University Hospital, were used.

Criteria for eligibility were that a primary tumor was histologically confirmed and that the subject was 20-years old or older. Patients with a history of other malignancies or who had diseases that could interfere with the analysis were excluded. Staging of gastric cancer was based on the 8th edition of the Union for International Cancer Control (UICC). Healthy adult individuals (N = 80) who underwent annual checkups (health examination, blood test, and chest X-ray) at Nagoya University Hospital and were not receiving any treatment for any disease were registered as the healthy control group. Serum samples (1 mL) were collected from volunteers or from patients prior to treatment (within 28 days before introduction of surgery, radiation therapy, or systemic therapy), and from patients who underwent R0 gastrectomy and had no recurrence of gastric cancer (within three months after the surgery). Blood collection was performed at an outpatient clinic between 8:00 AM and 12:00 PM, and the blood was collected into tubes containing a serum separation agent. The tubes were kept at 4°C for at least 30 minutes, centrifuged at 3,000 rpm for five minutes, and the serum was transferred to a new tube and stored at -80°C until the analysis.

Among the patients with gastric cancer, 275 were male and 121 were female, and the median age was 67.5 years (range, 28 to 92 years), and the mean age was 66.9 years (standard deviation, 12.5) (Table 1). Histological subtypes of gastric cancer were classified as differentiated (N = 175; papillary carcinoma, well differentiated adenocarcinoma, moderately differentiated adenocarcinoma) and undifferentiated (N = 221; poorly differentiated adenocarcinoma, signet ring cell carcinoma, mucinous adenocarcinoma). According to the classification of the UICC 8th edition, the numbers of patients with gastric cancer in Stages I, II, III, and IV were 184, 57, 68, and 87, respectively.

**[Table 1]**

| Variables | | Values |
|---|---|---|
| Age (years), mean ± standard deviation | | 66.9 ± 12.5 |
| Sex (male/female) | | 275/121 |
| Tumor location | | |
| | Upper third | 108 (27%) |
| | Middle third | 166 (42%) |
| | Lower third | 121 (31%) |
| Tumor size (mm), mean ± standard deviation | | 41.8 ± 28.1 |
| Differentiation | | |
| | Differentiated | 175 (44%) |
| | Undifferentiated | 221 (56%) |
| Clinical T factor | | |
| | T1 | 148 (37%) |
| | T2 | 102 (26%) |
| | T3 | 101 (26%) |
| | T4 | 45 (11%) |
| Clinical N factor | | |
| | NO | 234 (59%) |
| | N1 | 93 (23%) |
| | N2 | 51 (13%) |
| | N3 | 18 (5%) |
| Clinical stage | | |
| | I | 184 (47%) |
| | II | 57 (14%) |
| | III | 68 (17%) |
| | IV | 87 (22%) |

The SDF levels in the serum were measured using a human SDF4 ELISA kit (Novus Biologicals). It should be noted that clinical information of the patients was blinded during the ELISA assay. Each sample was tested in duplicate according to the protocol, and the average value was used. Cell lysates and conditioned media which will be described below were measured in the same manner.

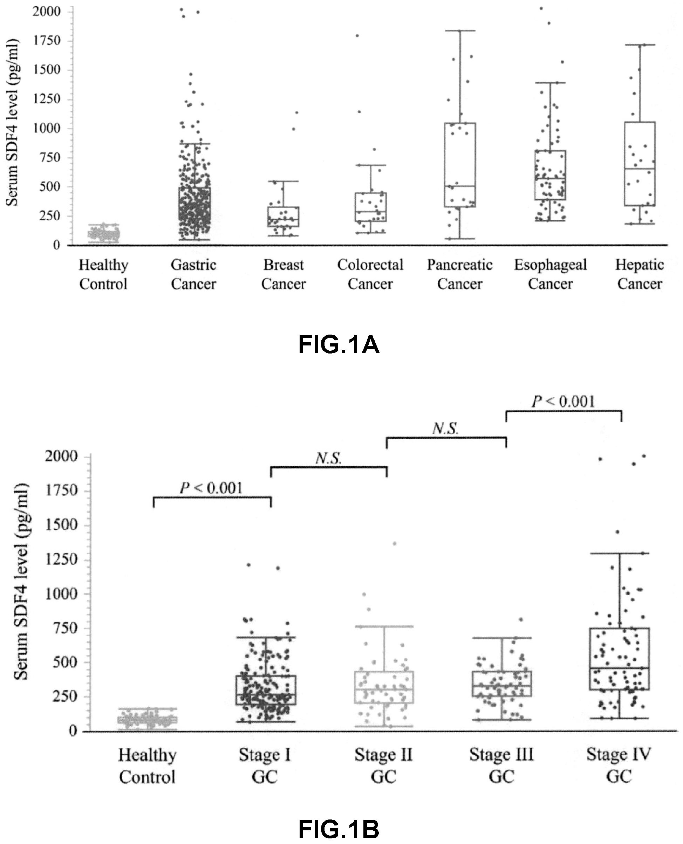

The median serum SDF4 level in 80 healthy individuals was 83.8 pg/mL (range, 17.9 to 169.9 pg/mL), whereas elevated SDF4 levels were observed in patients with any of the analyzed cancers, including gastric cancer, breast cancer, colorectal cancer, pancreatic cancer, esophageal cancer, and liver cancer (Figure 1A). Furthermore, the serum SDF4 levels in patients with gastric cancer tended to increase with the disease stage, with median values of 266.2 pg/mL (range, 70.4 to 1202 pg/mL) in Stage I, 303.4 pg/mL (range, 39.1 to 1353.1 pg/mL) in Stage II, 326.6 pg/mL (range, 84.1 to 808.8 pg/mL) in Stage III, and 455.1 pg/mL (range, 92.8 to 3185.3 pg/mL) in Stage IV (Figure 1B). The SDF4 levels in the patients with Stage I gastric cancer were significantly higher than those in the healthy individual group (P < 0.001), and the SDF4 levels in the patients with Stage IV gastric cancer were significantly higher than those in the patients with Stage III gastric cancer (P < 0.001). The significant difference in the SDF4 levels between the patients with Stage I gastric cancer and the healthy individuals demonstrates that patients with early-stage gastric cancer can be detected by blood testing.

Statistical analyses were performed using JMP 16 software (SAS Institute) as follows. Group means for categorical and continuous variables were compared using the chi-square test and the Mann-Whitney U test, respectively. Spearman's rank correlation coefficient was used to assess correlation between two continuous variables. P < 0.05 was considered statistically significant.

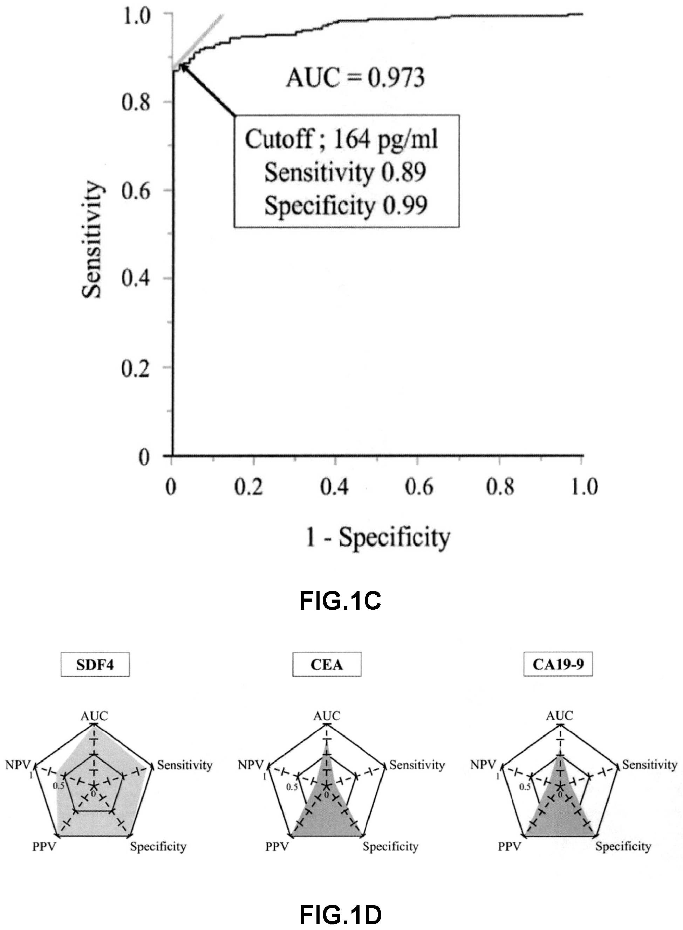

To determine the optimal cutoff value for distinguishing healthy individuals from patients with gastric cancer, receiver operating characteristic (ROC) analysis was performed. The analysis showed that the cutoff value of the SDF4 concentration for distinguishing healthy individuals from patients with gastric cancer was 164 pg/mL, with a sensitivity of 89% and a specificity of 99% (Figure 1C). Although the cutoff value may vary slightly depending on the antibody and detection reagents used, it can be appropriately set by measuring serum SDF4 levels in healthy individuals and the cancer patient group and performing the ROC analysis. The area under the curve (AUC) value for SDF4 was 0.973, which was higher than those of conventional cancer markers, CEA (0.75) and CA19-9 (0.639), demonstrating the superiority of the SDF4 as a diagnostic marker. Figure 1D summarizes the diagnostic performance of the SDF4, the CEA, and the CA19-9. Comparison of the AUC, the sensitivity, the specificity, a positive predictive value (PPV), and a negative predictive value (NPV) between the SDF4, the CEA, and the CA19-9, it was shown that the SDF4 had superior diagnostic performance to the CEA and the CA19-9.

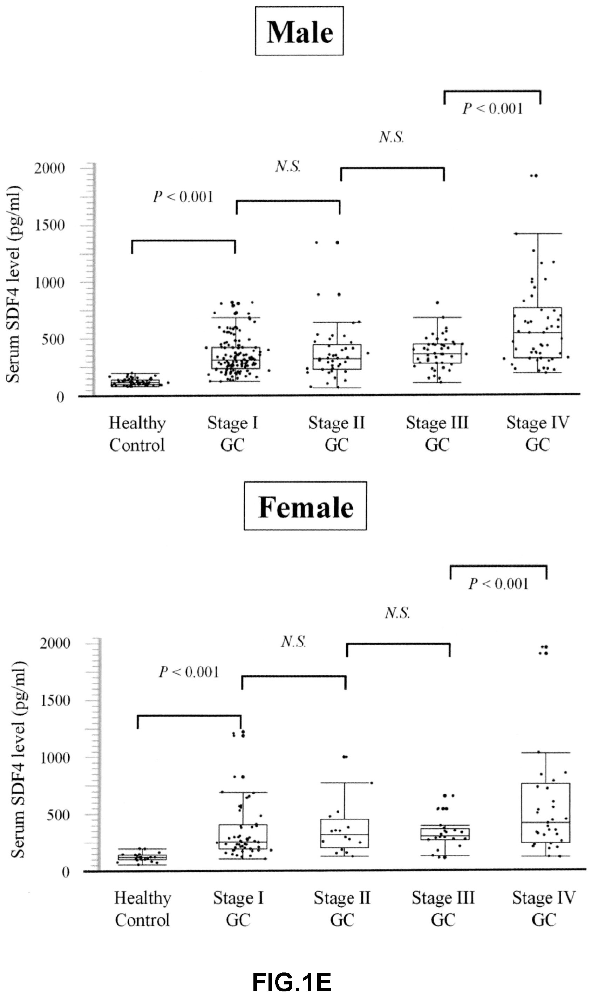

Furthermore, an analysis of serum SDF4 concentrations in the group of patients with gastric cancer, stratified by sex, was performed. The analysis for the SDF4 levels of each stage for each sex yielded results consistent with those from the entire cohort, with no sex-based differences observed (Figure 1E).

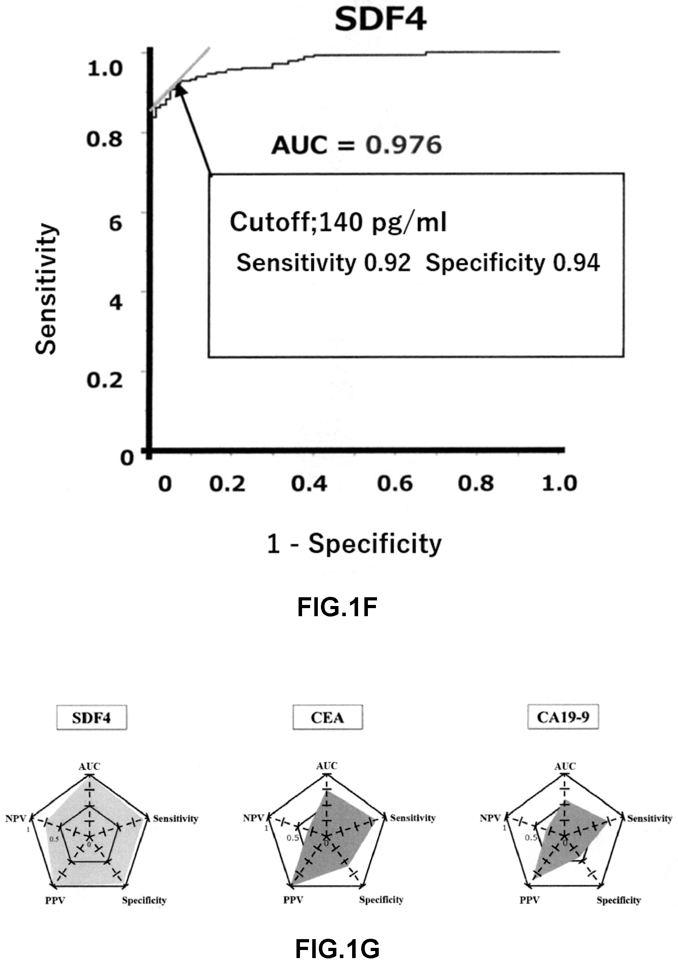

Next, the ROC analysis similar to that in Figure 1C, but limited to the patients with Stage I gastric cancer shown in Table 1, was performed. The cutoff value of the SDF4 concentration for distinguishing healthy individuals from the patients with gastric cancer was 140 pg/mL, with a sensitivity of 92%, a specificity of 94%, and an AUC of 0.976 (Figure 1F). Similarly, Figure 1G summarizes the diagnostic performance of the SDF4, the CEA, and the CA19-9 in the patients with Stage I gastric cancer. When comparing the AUC, the sensitivity, the specificity, the positive predictive value (PPV), and the negative predictive value (NPV), the SDF4 showed superior performance to the CEA and the CA19-9 in all parameters except the PPV, where it was comparable.

Thus, the SDF4 was shown to be a marker capable of specifically detecting even Stage I gastric cancer. In addition, it has been demonstrated that a predetermined value can be set based on the above results to enable diagnosis.

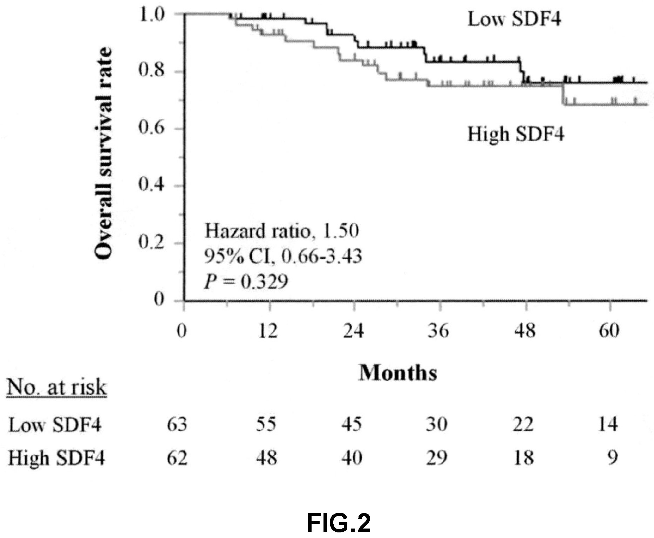

Next, the present inventor analyzed whether the serum SDF4 level was also useful as a prognostic marker in a patient with gastric cancer. Overall survival (OS) was estimated using the Kaplan-Meier method with the log-rank test, and was defined as an interval from the date of curative gastrectomy to death. The OS was compared in patients with advanced gastric cancer in the cohort. Patients with Stage II gastric cancer and Stage III gastric cancer (N = 125) were divided into two groups based on the median serum SDF4 level of 313.7 pg/mL (low SDF4 group, N = 63; high SDF4 group, N = 62). Comparison of the OS between the two groups showed no significant difference (hazard ratio, 1.50; 95% confidence interval, 0.66 to 3.43; P = 0.329) (Figure 2). The rates of postoperative adjuvant chemotherapy were 54.84% (34 in 62 cases) in the high SDF4 group and 57.14% (36 in 63 cases) in the low SDF4 group, with no significant difference between the groups (P = 0.795). These findings indicate that although the SDF4 level can serve as a marker for detecting cancer, they do not function as a prognostic marker.

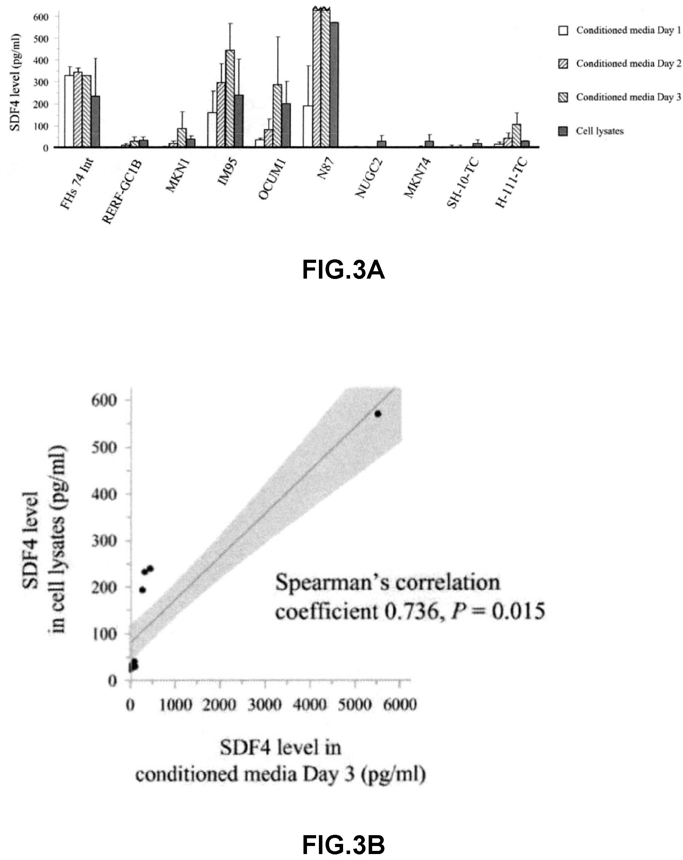

Next, in order to determine whether circulating SDF4 in the blood is derived from a gastric cancer cell, the present inventor measured the SDF4 concentrations in cell lysates and conditioned media of nine gastric cancer cell lines and the normal intestinal epithelial cell line FHs 74 Int by ELISA (Figure 3A). The nine gastric cancer cell lines (RERF-GC-1B, MKN1, IM95, OCUM1, N87, NUGC2, MKN74, SH-10-TC, and H-111-TC) and the normal intestinal epithelial cell line FHs 74 Int were obtained from ATCC or the JCRB Cell Bank. The cell lysates were prepared by washing cultured cells (10⁶ cells) twice with PBS and then lysing them in a RIPA buffer (Thermo Fisher Scientific). The conditioned media were obtained by culturing 10 mL of gastric cancer cells or intestinal epithelial cells (10⁵ cells/mL) in a cell culture dish with a diameter of 10 cm in RPMI 1640 medium containing 10% fetal bovine serum. After culturing each cell line in separate dishes for one, two, or three days, the culture supernatants were collected, centrifuged at 3000 rpm for five minutes, stored at -30°C, and then used for analysis.

Several gastric cancer cell lines secreted the SDF4 during the culture period. A significant correlation was detected between the intracellular SDF4 level (the SDF4 level in the cell lysates) and the secreted level (the SDF4 level in the conditioned media) on the day 3 of culture (Figures 3A and 3B; Spearman's correlation coefficient: 0.736; P = 0.015).

The results of the experiments using the cell lines indicate that human gastric cancer cell lines not only express the SDF4 protein but also secrete it into the culture medium. Since the level of the secreted SDF4 correlated positively with the intracellular SDF4 level, the elevated serum SDF4 levels are suggested to result from active secretion by viable cells or passive release from gastric cancer cells which underwent apoptosis. As described above, the SDF4 has three splice variants, one of which is a secreted form. Elevated concentrations of the SDF4 in the serum of patients with cancer may thus be attributable to an increase in the secreted SDF4 isoform caused by the splicing or a passive increase resulting from apoptosis.

In addition, some gastric cancer cell lines produced little SDF4 in vitro (Figure 3A). Since this analysis was based on experiments using cell lines, it may reflect the heterogeneity of the gastric cancer tissue. It is also possible that in the patients with gastric cancer, the serum SDF4 levels were elevated due to the secretion from other organs. For example, the elevated serum SDF4 concentrations in the patients with cancer may originate not only from cancer cells but also from cells other than the cancer cells, which were activated under inflammatory conditions within the tumor microenvironment and from normal tissues. It is possible that as a result the significant increase in the serum SDF4 levels as compared to those in the healthy individuals have been observed in the patients with early-stage gastric cancer. Furthermore, the SDF4 was also secreted by the normal intestinal epithelial cell line FHs 74 Int, which is considered to be consistent with the presence of a certain amount of SDF4 in the blood of the healthy individuals. This suggests that the production of the SDF4 by the cell lines does not necessarily result directly in high serum concentrations.

Expression of the SDF4 protein in a gastric cancer tissue and changes in expression patterns according to stage were analyzed by an immunohistochemical method. Immunohistochemical staining of the SDF4 protein was performed by incubating tissue sections with a rabbit polyclonal anti-SDF4 antibody (Funakoshi) diluted to 1/500 in antibody diluent (Dako) for one hour at room temperature, followed by incubation with a biotinylated secondary antibody (HRP-labeled SignalStain (trademark) Boost IHC Detection Reagent, Cell Signaling Technology) for 30 minutes, and then visualizing the antigen-antibody complex by exposing the sections to liquid 3,3'-diaminobenzidine (NICHIREI) for one minute.

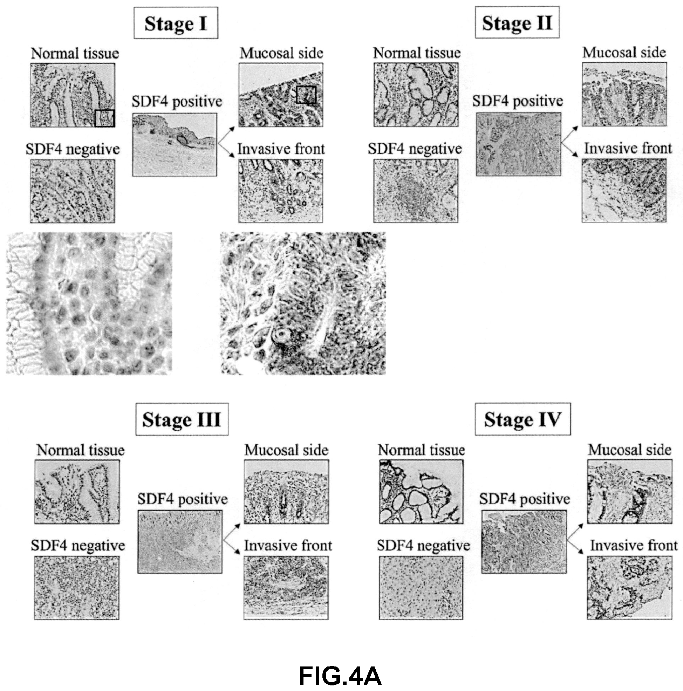

The immunohistochemical staining was performed on tissues affected with gastric cancer of Stage I to IV and adjacent normal tissues (Figure 4A). In Figure 4A, enlarged views of the boxed areas in normal tissue (Normal tissue) and tumor tissue (Mucosal side) at Stage I are shown in a lower part of the drawing. No expression of the SDF4 was observed in the normal tissues (positive staining rate: 0%). In gastric cancer tissue sections, the SDF4 was observed in the cytoplasm of the cancer cells, but was not observed in the tumor stroma. In the SDF4-positive tumor tissues, positive staining was observed on both the mucosal side and the invasive front.

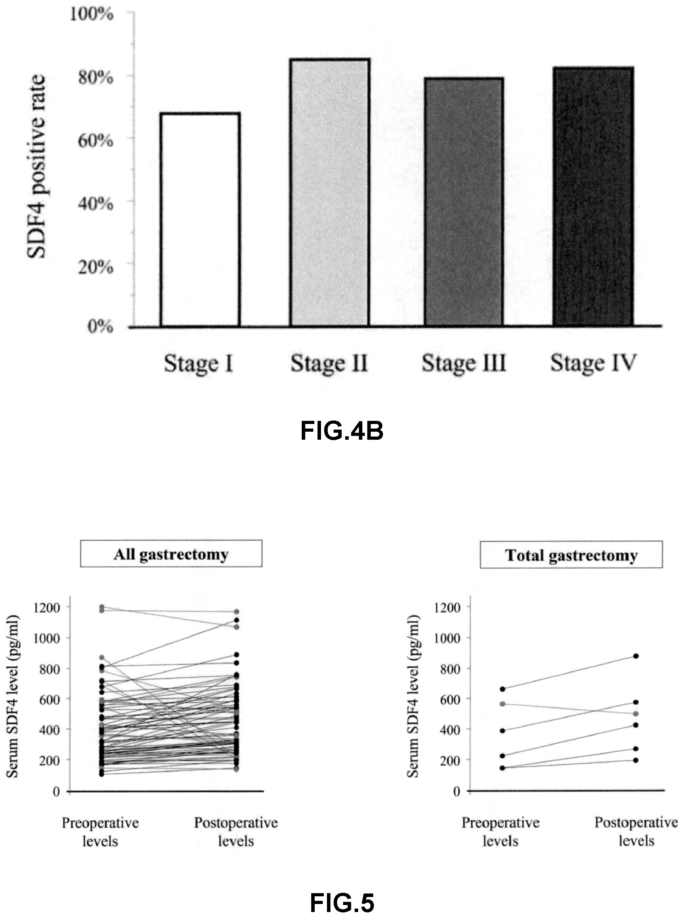

Most tissue samples from all disease stages were positive for the SDF4, and no significant differences in the positive staining rate were observed among the disease stages (Stage I: 66.7%, N = 18; Stage II: 84.6%, N = 13; Stage III: 78.6%, N = 14; Stage IV: 81.8%, N = 11) (Figure 4B).

Next, preoperative serum samples were compared with samples obtained within three months after R0 gastrectomy in 73 patients without recurrence of gastric cancer to examine whether the serum SDF4 levels change during the perioperative period. Since an acute inflammatory condition is considered to subside within three months, the SDF4 levels were assumed not to be affected by the inflammatory condition. Further, also for patients who received the total gastrectomy, the serum SDF4 levels before and after the total gastrectomy were compared. In patients who underwent resection of the primary gastric cancer lesion (Figure 5, left) or in six patients who underwent total gastrectomy (Figure 5, right), no significant changes in the serum SDF4 levels were observed postoperatively.

The absence of changes in the SDF4 levels before and after the surgery suggests that the half-life of the SDF4 in the blood is very long, and that it may not be cleared from circulating body fluids for at least three months after the tumor resection. Alternatively, the production of the SDF4 in normal tissues such as the liver may be elevated due to the postoperative inflammation and induction of inflammatory cytokines, leading to the secretion of the SDF4 from tissues other than the cancer tissues. This contrasts with the CEA, which has an average half-life of approximately ten days, and suggests that the serum SDF level is not a marker for recurrence of gastric cancer after a curative surgery.

As shown in Figures 1A to 1G, the characteristic feature of the SDF4 as a diagnostic marker is that it can detect cancer, including even early-stage cancer, using a blood sample. By using an immunoassay which is widely used in clinical settings, such as ELISA, it is possible to detect cancer with a high sensitivity and specificity. Examples of the cancer that can be detected using the SDF4 include gastric cancer, breast cancer, colorectal cancer, pancreatic cancer, esophageal cancer, and liver cancer, as shown in Figure 1A.

Furthermore, by using a public gene expression database (GENT2) for comparing gene expression between a cancer tissue and a normal tissue, it may be possible to detect liposarcoma, bladder cancer, brain tumors, head and neck cancer, gallbladder cancer, ovarian cancer, tongue cancer, and uterine cancer, where elevated expression of the SDF4 is observed in cancer tissues as compared to that in normal tissues. As demonstrated in the experimental results presented above, the SDF4 circulating in the blood is considered to be a secreted form or released into the blood due to apoptosis. Therefore, it is highly likely that cancer in which the SDF4 expression is increased in cancer tissue can also be detected. A biomarker that can detect a wide variety of early-stage cancer types with a high sensitivity and specificity has not been reported so far. Cancer testing using the SDF4 is believed to contribute to the detection of early-stage cancers. In other words, by using the SDF4 as a diagnostic marker, it is possible to determine a subject as being likely to have any of various cancers, for example, gastric cancer, breast cancer, colorectal cancer, pancreatic cancer, esophageal cancer, liver cancer, liposarcoma, bladder cancer, brain tumors, head and neck cancer, gallbladder cancer, ovarian cancer, tongue cancer, and uterine cancer, and more specifically gastric cancer, breast cancer, colorectal cancer, pancreatic cancer, esophageal cancer, and liver cancer. This enables earlier implementation of detailed examinations and early diagnosis and treatment of cancer.

## Claims

1. A test support method including measuring a concentration of SDF4 in a body fluid of a subject, and determining the subject as having cancer when the concentration is higher than a predetermined value.

2. The method according to claim 1, wherein the subject is identified as having cancer using a criterion that the subject is highly likely to have cancer when a level of the SDF4 in the body fluid is higher than the level in a healthy individual.

3. The method according to claim 1, wherein the body fluid is blood.

4. The method according to claim 3, wherein the blood is selected from whole blood, serum, and plasma.

5. The method according to claim 1, wherein the concentration of the SDF4 in the body fluid is measured by an immunoassay.

6. The method according to claim 1, wherein the cancer is gastric cancer, breast cancer, colorectal cancer, pancreatic cancer, esophageal cancer, liver cancer, liposarcoma, bladder cancer, brain tumor, head and neck cancer, gallbladder cancer, ovarian cancer, tongue cancer, or uterine cancer.

7. The method according to any one of claims 1 to 6, wherein the cancer is early-stage cancer.

8. The method according to claim 7, wherein the early-stage cancer is early-stage gastric cancer.

9. A method of acquiring data for diagnosing cancer, the method including a step of measuring a concentration of SDF4 in a body fluid of a subject.

10. The method according to claim 9, wherein the body fluid is blood and is specifically selected from whole blood, serum, and plasma.

11. A cancer marker including SDF4 present in a body fluid, the marker exhibiting upregulated expression in a body fluid of a patient with cancer and serving as a cancer diagnostic marker.

12. A test kit for detecting cancer using a body fluid of a subject as a sample, the kit including a reagent that specifically binds to SDF4 and a detection reagent.

13. The test kit according to claim 12, wherein the cancer is gastric cancer, breast cancer, colorectal cancer, pancreatic cancer, esophageal cancer, liver cancer, liposarcoma, bladder cancer, brain tumor, head and neck cancer, gallbladder cancer, ovarian cancer, tongue cancer, or uterine cancer.

14. The test kit according to claim 12 or 13, wherein the reagent that specifically binds to the SDF4 is an antibody.

15. The test kit according to claim 14, wherein the detection is performed by ELISA.
